# DEMANDE DE BREVET EUROPEEN

(11) **EP 2 103 937 A1**
(43) Date de publication de la demande: **23.09.2009**
(21) Numéro de dépôt: 09290200.6
(22) Date de dépôt: 18.03.2009
(51) Int. Cl.: G01N 33/569, G01N 33/58, G01N 33/533

(54) **Procédé d'analyse de cellules par cytométrie en flux, utilisation d'un agent antioxydant et/ou piégeant les radicaux libres dans un tel procédé et tampon d'incubation comprenant un agent antioxydant et/ou piégeant les radicaux libres**

(30) Priorité: 20.03.2008 WO PCT/FR2008/000380
(71) Demandeur: Assistance Publique - Hôpitaux de Paris, 75004 Paris (FR)
(72) Inventeur: Letestu, Rémi, 93160 Noisy Le Grand (FR); Le Roy, Christine, 75019 Paris (FR); Cymbalista, Florence, 75011 Paris (FR); Varin-Blank, Nadine, 78500 Sartrouville (FR)
(74) Mandataire: Noel, Chantal Odile

(57) **Abrégé**

L'invention concerne un procédé d'analyse de cellules par cytométrie en flux ainsi que l'utilisation d'un agent antioxydant et/ou piégeant les radicaux libres dans un tel procédé. Elle concerne également un tampon d'incubation comprenant un agent antioxydant et/ou piégeant les radicaux libres.

Le procédé de l'invention comprend une étape de marquage des cellules dans une solution de marquage comprenant un tampon d'incubation et le ou les anticorps de marquage conjugués à au moins un fluorochrome qui est un tandem APC et un agent antioxydant et/ou piégeant les radicaux libres.

L'invention trouve application dans le domaine de l'analyse de cellules par cytométrie en flux, en particulier.

## Description

L'invention concerne un procédé d'analyse de cellules par cytométrie en flux ainsi que l'utilisation d'un agent antioxydant et/ou piégeant les radicaux libres dans un tel procédé. Elle concerne également un tampon d'incubation comprenant un agent antioxydant et/ou piégeant les radicaux libres.

La méthodologie de cytométrie en flux permet la mesure simultanément de paramètres morphologiques et fluorescents distincts au niveau de la cellule. Le développement de systèmes multi-couleurs a de nombreux avantages comme de fournir une grande quantité d'informations au niveau de la cellule elle-même, une mesure précise même dans des populations cellulaires rares, et identification de nouvelles caractéristiques cellulaires.

En particulier, cette technologie est cruciale pour l'identification fonctionnelle de sous-populations de cellules dans le système hématopoïétique entier, permettant une meilleure compréhension de la physiologie de la cellule et de la physiopathologie des pathologies malignes.

Elle est donc régulièrement utilisée en hématologie pour le diagnostic ou le suivi thérapeutique de différentes affections où elle permet la détection de cellules sanguines pathologiques et leur différenciation par rapport aux cellules sanguines saines.

Comme les expériences de cytométrie en flux deviennent de plus en plus complexes, la demande pour de nouveaux fluorochromes avec différentes propriétés augmente.

Les fluorochromes (colorants fluorescents) absorbent la lumière à des longueurs d'ondes données et à leur tour émettent leur énergie de fluorescence à une longueur d'onde plus élevée.

Parmi les nouveaux fluorochromes, le tandem APC-Cy7 (allophycocyanine-cyanine 7) et son analogue APC-H7 (allophycocyanine-Hilite^{®}7-BD) sont des fluorochromes appelés tandems APC présentant des propriétés spectrales similaires avec un maximum d'absorption à approximativement 650 nm. Sous une excitation provenant d'un laser rouge (HeNe, 633 nm), le fluorochrome APC présent dans le tandem APC transfère son énergie au colorant cyanine, qui à son tour émet à 767 nm. Les fluorochromes tandems APC sont conjugués à des anticorps dirigés contre des antigènes cellulaires.

Cependant, l'excitation de ces conjugués anticorps-tandems APC peut résulter en une émission dans le canal APC. Cette émission, communément observée par les cytométristes en flux, mène à des évènements faux positifs et à une sensibilité réduite du marquage.

Jusqu'à présent, l'émission dans le canal APC était expliquée par au moins deux mécanismes. D'abord, une association non optimale du colorant APC et des colorants Cy7 ou H7 peut mener à une émission de fluorescence de l'APC reflétant une réabsorption incomplète par les colorants Cy7 ou H7 de la fluorescence émise par la molécule d'APC. Deuxièmement, un manque de stabilité des tandems APC-Cy7 et APC-H7 peut résulter en un signal APC "parasite". Ce mécanisme est généralement appelé "phénomène de découplage".

Le manque de stabilité des tandems APC (c'est-à-dire des tandems de colorants dont l'un est l'APC) est généralement attribué à une photo- dégradation. En effet, il est connu que les fluorochromes sont sensibles à la lumière. C'est pourquoi les analyses, incluant la préparation des cellules à analyser (lyse, immuno-marquage etc...) sont effectuées à l'abri de la lumière, ce qui est une précaution particulièrement mise en avant pour les conjugués comprenant les tandems APC.

Par ailleurs, l'acide ascorbique est un antioxydant capable de contrer l'action néfaste des oxydants et des radicaux libres.

La forme L de l'acide ascorbique, la vitamine C, a de plus une activité vitaminique.

Cependant, la vitamine C est très fragile en solution, elle est détruite au contact de l'air, par la lumière ou la chaleur.

L'invention a pour but de proposer des mesures permettant d'effectuer des analyses de cellules par cytométrie en flux en utilisant, en tant que fluorochromes, les tandems APC sans que ne se produise le phénomène de découplage ou qu'au moins ce dernier soit inhibé.

A cet effet, l'invention propose un procédé d'analyse de cellules par cytométrie en flux comprenant une étape de marquage des cellules par incubation dans une solution de marquage comprenant un tampon d'incubation et des anticorps de marquage conjugués à au moins un fluorochrome qui est un tandem APC, **caractérisé en ce qu**'au moins un agent antioxydant et/ou piégeant les radicaux libres, est introduit dans la solution de marquage.

Par tandem APC, on entend un tandem APC-Cy7, un tandem APC-H7 ou un tandem APC-Alexa 750.

De préférence, les cellules sont des cellules sanguines.

Toujours de préférence, l'agent antioxydant, et/ou piégeant les radicaux libres, est l'acide ascorbique.

Le plus préférablement, l'agent antioxydant et/ou piégeant les radicaux libres est la vitamine C.

Lorsque l'agent antioxydant et/ou piégeant les radicaux libres, est la vitamine C, et lorsque les cellules sont des lymphocytes, la vitamine C est présente dans la solution analysée par cytométrie en flux à une concentration finale comprise entre 0,1mM et 10 mM inclus, de préférence à une concentration finale comprise entre 1 mM et 10 mM inclus, le plus préférablement à une concentration de 1 mM.

Lorsque l'agent antioxydant, et/ou piégeant les radicaux libres est la vitamine C et que les cellules sont des monocytes, la vitamine C est présente dans la solution analysée par cytométrie en flux à une concentration finale comprise entre 1 mM et 10 mM inclus, le plus préférablement à une concentration de 1 mM.

Lorsque l'agent antioxydant, et/ou piégeant les radicaux libres est la vitamine C et que les cellules analysées sont des cellules polynucléaires neutrophiles, la vitamine C est présente à une concentration finale dans la solution analysée comprise entre 0,25 mM et 10 mM inclus, de préférence comprise entre 0,75 mM et 4 mM inclus, plus préférablement comprise entre 0,75 mM et 4 mM inclus, le plus préférablement de 0,75 Mm.

L'invention propose aussi l'utilisation d'un agent antioxydant et/ou piégeant les radicaux libres, pour inhiber la dégradation des fluorochromes tandems APC-Cy7, APC-H7 ou APC-Alexa 750 lors de l'analyse de cellules par cytométrie en flux.

De préférence, dans l'utilisation de l'invention, les cellules sont des cellules sanguines.

Également de préférence, dans l'utilisation de l'invention, l'agent antioxydant et/ou piégeant les radicaux libres, est l'acide ascorbique, le plus préférablement sous sa forme L (vitamine C).

Lorsque l'agent antioxydant et/ou piégeant les radicaux libres, est la vitamine C, et lorsque les cellules sont des lymphocytes, la vitamine C est présente dans la solution analysée par cytométrie en flux à une concentration finale comprise entre 0,1mM et 10 mM inclus, de préférence à une concentration finale comprise entre 1 mM et 10 mM inclus, le plus préférablement à une concentration de 1 mM.

Lorsque l'agent antioxydant, et/ou piégeant les radicaux libres est la vitamine C et que les cellules sont des monocytes, la vitamine C est présente dans la solution analysée par cytométrie en flux à une concentration finale comprise entre 1 mM et 10 mM inclus, le plus préférablement à une concentration de 1 mM.

Lorsque l'agent antioxydant, et/ou piégeant les radicaux libres est la vitamine C et que les cellules analysées sont des cellules polynucléaires neutrophiles, la vitamine C est présente à une concentration finale dans la solution analysée comprise entre 0,25 mM et 10- mM inclus, de préférence comprise entre 0,75 mM et 4 mM inclus, plus préférablement comprise entre 0,75 mM et 4 mM inclus, le plus préférablement de 0,75 mM.

L'invention propose également un tampon d'incubation comprenant un agent antioxydant et/ou piégeant les radicaux libres.

De préférence, dans le tampon d'incubation de l'invention, l'agent antioxydant, et/ou piégeant les radicaux libres, est l'acide ascorbique, plus préférablement sous sa forme L, la vitamine C.

L'invention sera mieux comprise et d'autres caractéristiques et avantages de celle-ci apparaîtront plus clairement à la lecture de la description explicative qui suit et qui est faite en référence aux figures dans lesquelles :
- la figure 1 est une image provenant du cytomètre montrant l'intensité de fluorescence, en unités arbitraires, (abscisse) de cellules polynucléaires neutrophiles, de monocytes et de lymphocytes en fonction de leur granularité, en unités arbitraires, marqués par des anticorps anti-CD45 (α-CD45) conjugués au tandem APC-Cy7,
- la figure 2 montre l'intensité du signal de fluorescence de lymphocytes marqués par des anticorps anti-CD45 conjugués au tandem APC-Cy7 dans le canal APC et dans le canal APC-Cy7 juste après incubation (T0),
- la figure 3 montre l'intensité du signal de fluorescence de lymphocytes marqués par des anticorps anti-CD45 conjugués au tandem APC-Cy7 dans le canal APC et dans le canal APC-Cy7 30 minutes après incubation (T30),
- la figure 4 montre l'évolution du pourcentage de découplage observé au temps T0 et T30 de lymphocytes marqués par des anticorps anti-CD45 conjugués au tandem APC-Cy7,
- la figure 5 est une image provenant du cytomètre montrant l'intensité de fluorescence, en unités arbitraires, (abscisse) de cellules polynucléaires neutrophiles, de monocytes et de lymphocytes en fonction de leur granularité, en unités arbitraires, marqués par des anticorps anti-CD45 (α-CD45) conjugués au tandem APC-H7,
- la figure 6 montre l'intensité du signal de fluorescence de lymphocytes marqués par des anticorps anti-CD45 conjugués au tandem APC-H7 dans le canal APC et dans le canal APC-H7 juste après incubation (T0),
- la figure 7 montre l'intensité du signal de fluorescence de lymphocytes marqués par des anticorps anti-CD45 conjugués au tandem APC-H7 dans le canal APC et dans le canal APC-H7 30 minutes après incubation (T30),
- la figure 8 montre l'évolution du pourcentage de découplage observé au temps T0 et T30 de lymphocytes marqués par des anticorps anti-CD45 conjugués au tandem APC-H7,
- la figure 9 est une représentation schématique du mode de liaison des anticorps conjugués aux tandems APC à des billes Compbeads^{®} de Becton Dickinson et à des cellules,
- la figure 10 montre l'évolution du pourcentage de découplage, en fonction du temps, observé lors de l'analyse par cytométrie en flux de billes Compbeads^{®} de Becton Dickinson marquées par un anticorps anti-CD45 conjugué au tandem APC-Cy7 et de lymphocytes marqués par un anticorps anti-CD45 conjugué au tandem APC-Cy7,
- la figure 11 montre l'évolution du pourcentage de découplage, en fonction du temps, observé lors de l'analyse par cytométrie en flux de billes (billes Compbeads^{®} de Becton Dickinson) marquées par un anticorps anti-CD45 conjugué au tandem APC-H7 et de lymphocytes marqués par un anticorps anti-CD45 conjugué au tandem APC-H7,
- la figure 12 montre l'évolution du pourcentage de découplage, en fonction du temps, observé lors de l'analyse par cytométrie en flux de lymphocytes marqués par un anticorps anti-CD20 (α-CD20) conjugué au tandem APC-Cy7, et de lymphocytes marqués par un anticorps anti-CD20 conjugué au tandem APC-H7,
- la figure 13 montre l'évolution du pourcentage de découplage, en fonction du temps, observé lors de l'analyse par cytométrie en flux de lymphocytes marqués par un anticorps anti-CD3 (α-CD3) conjugué au tandem APC-Cy7, et de lymphocytes marqués par un anticorps anti-CD3 conjugué au tandem APC-H7,
- la figure 14 montre l'évolution du pourcentage de découplage, en fonction du temps, observé lors de l'analyse par cytométrie en flux de lymphocytes marqués par un anticorps anti-CD45 conjugué au tandem APC-Cy7 ou au tandem APC-H7, et de cellules polynucléaires neutrophiles marquées par anticorps anti-CD45 conjugué au tandem APC-Cy7 ou au tandem APC-H7,
- la figure 15 montre l'évolution du pourcentage de découplage, en fonction du temps, observé lors de l'analyse par cytométrie en flux de monocytes marqués par un anticorps anti-CD45 conjugué au tandem APC-Cy7 ou au tandem APC-H7,
- la figure 16 montre l'évolution du pourcentage de découplage, en fonction du temps, observé lors de l'analyse par cytométrie en flux de :
   a) lymphocytes marqués par un anticorps anti-CD45 conjugué au tandem APC-Cy7, à l'abri de la lumière,
   b) lymphocytes marqués par un anticorps anti-CD45 conjugué au tandem APC-Cy7, avec exposition à la lumière,
   c) monocytes marqués par un anticorps anti-CD45 conjugué au tandem APC-Cy7, à l'abri de la lumière,
   d) monocytes marqués par un anticorps anti-CD45 conjugué au tandem APC-Cy7, avec exposition à la lumière,
   e) cellules polynucléaires neutrophiles marquées par un anticorps anti-CD45 conjugué au tandem APC-Cy7, à l'abri de la lumière,
   f) cellules polynucléaires neutrophiles marquées par un anticorps anti-CD45 conjugué au tandem APC-Cy7, avec exposition à la lumière,

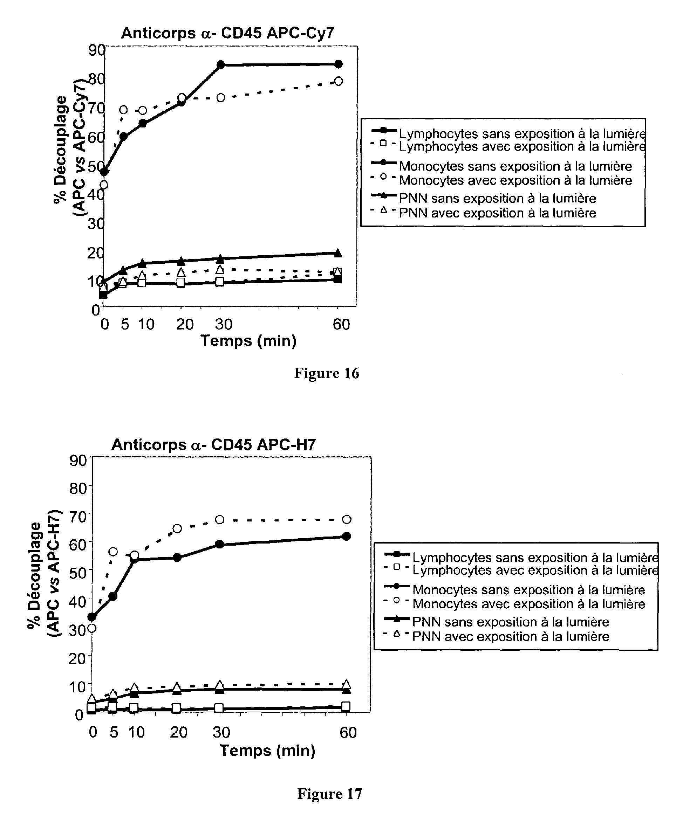

- la figure 17 montre l'évolution du pourcentage de découplage, en fonction du temps, observé lors de l'analyse par cytométrie en flux de :
   a) lymphocytes marqués par un anticorps anti-CD45 conjugué au tandem APC-H7, à l'abri de la lumière,
   b) lymphocytes marqués par un anticorps anti-CD45 conjugué au tandem APC-H7, avec exposition à la lumière,
   c) monocytes marqués par un anticorps anti-CD45 conjugué au tandem APC-H7, à l'abri de la lumière,
   d) monocytes marqués par un anticorps anti-CD45 conjugué au tandem APC-H7, avec exposition à la lumière,
   e) cellules polynucléaires neutrophiles marquées par un anticorps anti-CD45 conjugué au tandem APC-H7, à l'abri de la lumière,
   f) cellules polynucléaires neutrophiles marquées par un anticorps anti-CD45 conjugué au tandem APC-H7, avec exposition à la lumière,

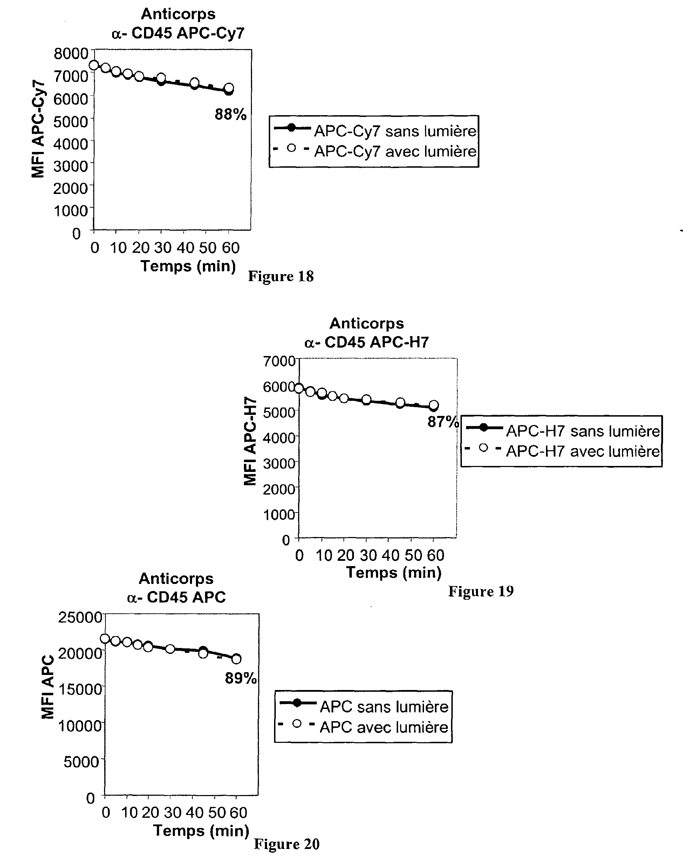

- la figure 18 montre l'évolution de l'intensité moyenne de fluorescence, en fonction du temps de billes (billes Compbeads^{®} de Becton Dickinson) marquées par un anticorps anti-CD45 conjugué au tandem APC-Cy7 en présence et en l'absence de lumière,
- la figure 19 montre l'évolution de l'intensité moyenne de fluorescence, en fonction du temps de billes (billes Compbeads^{®} de Becton Dickinson) marquées par un anticorps anti-CD45 conjugué au tandem APC-H7 en présence et en l'absence de lumière,
- la figure 20 montre l'évolution de l'intensité moyenne de fluorescence, en fonction du temps de billes (billes Compbeads^{®} de Becton Dickinson) marquées par un anticorps anti-CD45 conjugué au fluorochrome APC en présence et en l'absence de lumière,

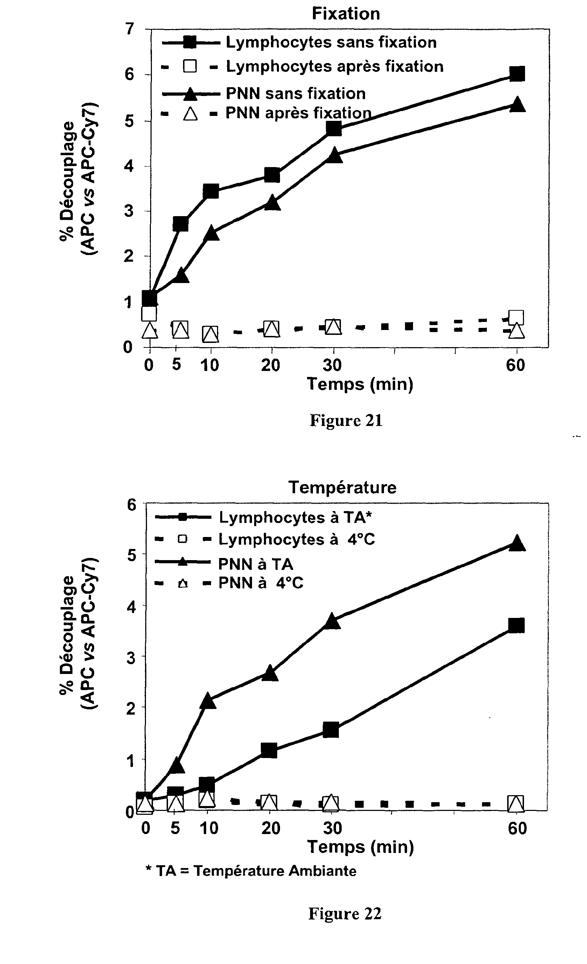

- la figure 21 montre l'évolution du pourcentage de découplage, en fonction du temps, observé lors de l'analyse par cytométrie en flux de :
   a) lymphocytes marqués par un anticorps anti-CD45 conjugué au tandem APC-Cy7, non fixés,
   b) lymphocytes marqués par un anticorps anti-CD45 conjugué au tandem APC-Cy7, fixés,
   c) cellules polynucléaires neutrophiles marquées par un anticorps anti-CD45 conjugué au tandem APC-Cy7, non fixées,
   d) cellules polynucléaires neutrophiles marquées par un anticorps anti-CD45 conjugué au tandem APC-Cy7, fixées,
- la figure 22 montre l'évolution du pourcentage de découplage, en fonction du temps, observé lors de l'analyse par cytométrie en flux de :
   a) lymphocytes marqués par un anticorps anti-CD45 conjugué au tandem APC-Cy7, incubés à température ambiante,
   b) lymphocytes marqués par un anticorps anti-CD45 conjugué au tandem APC-Cy7, incubés à 4°C,
   c) cellules polynucléaires neutrophiles marquées par un anticorps anti-CD45 conjugué au tandem APC-Cy7, incubées à température ambiante,
   d) cellules polynucléaires neutrophiles marquées par un anticorps anti-CD45 conjugué au tandem APC-Cy7, incubées à 4°C,

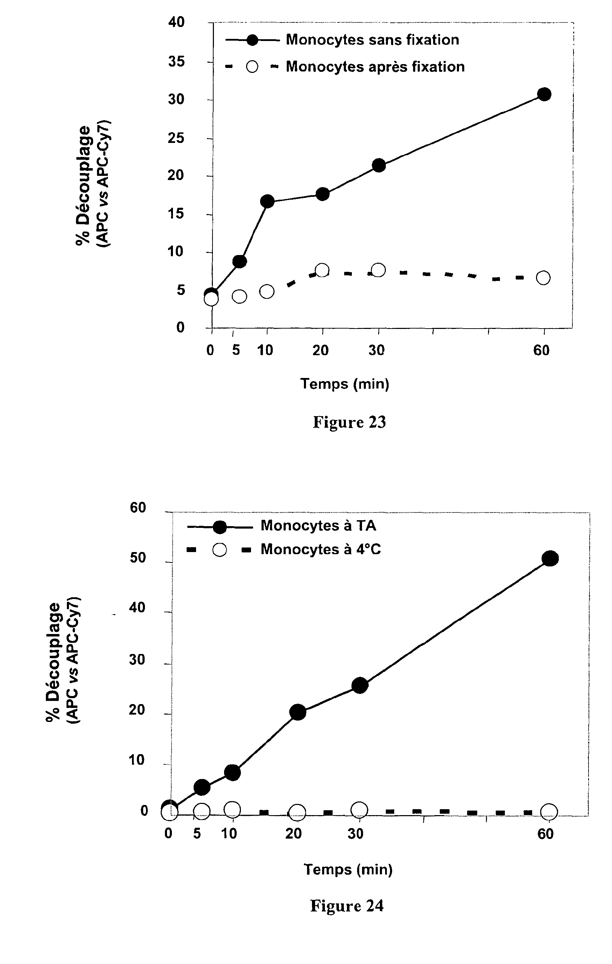

- la figure 23 montre l'évolution du pourcentage de découplage, en fonction du temps, observé lors de l'analyse par cytométrie en flux de monocytes marqués par un anticorps anti-CD45 conjugué au tandem APC-Cy7, non fixés, et de monocytes marqués par un anticorps anti-CD45 conjugué au tandem APC-Cy7, fixés,
- la figure 24 montre l'évolution du pourcentage de découplage, en fonction du temps, observé lors de l'analyse par cytométrie en flux de monocytes marqués par un anticorps anti-CD45 conjugué au tandem APC-Cy7, incubés à température ambiante, et de monocytes marqués par un anticorps anti-CD45 conjugué au tandem APC-Cy7, incubés à 4°C,

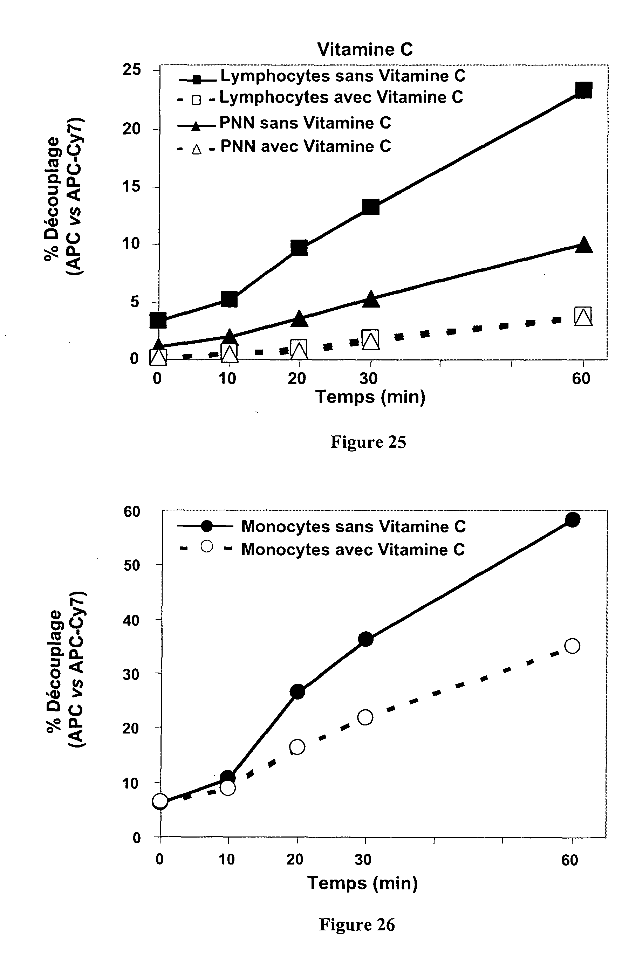

- la figure 25 montre l'évolution du pourcentage de découplage, en fonction du temps, observé lors de l'analyse par cytométrie en flux de :
   a) lymphocytes marqués par un anticorps anti-CD45 conjugué au tandem APC-Cy7, incubés sans vitamine C,
   b) lymphocytes marqués par un anticorps anti-CD45 conjugué au tandem APC-Cy7, incubés en présence de vitamine C 1 mM,
   c) cellules polynucléaires neutrophiles marquées par un anticorps anti-CD45 conjugué au tandem APC-Cy7, incubées sans vitamine C,
   d) cellules polynucléaires neutrophiles marquées par un anticorps anti-CD45 conjugué au tandem APC-Cy7, incubées en présence de vitamine C 1mM, et
- la figure 26 montre l'évolution du pourcentage de découplage, en fonction du temps, observé lors de l'analyse par cytométrie en flux de monocytes marqués par un anticorps anti-CD45 conjugué au tandem APC-Cy7, incubés sans vitamine C, et de monocytes marqués par un anticorps anti-CD45 conjugué au tandem APC-Cy7, incubés en présence de vitamine C 1mM.

Les termes « pourcentage de découplage », « % découplage », « % découplage APC vs APC-Cy7 », « % découplage APC vs APC-H7 » sont utilisés dans le texte qui précède et qui suit et dans les figures annexées pour désigner le rapport :

[Nombre de cellules qui, bien qu'ayant été marquées par un tandem APC émettent une fluorescence dans le canal APC (et non dans le canal du tandem APC utilisé)] / [Nombre total de cellules qui ont été marquées par le tandem APC, quelque soit le canal dans lequel elles émettent],
ce rapport étant multiplié par 100.

Les termes "température ambiante" sont utilisés dans le texte qui précède et qui suit ainsi que dans les figures annexées pour désigner une température comprise entre 18°C et 25°C inclus.

L'invention repose sur l'élucidation par les inventeurs du mécanisme d'altération des marqueurs conjugués avec les tandems APC sur des cellules immuno-marquées, qui mène au phénomène de découplage constaté lors de l'analyse de ces cellules par cytométrie en flux en utilisant comme fluorochromes les tandems APC.

Pour élucider le mécanisme menant au phénomène de découplage, les inventeurs ont analysé par cytométrie en flux des échantillons de cellules marquées par différents anticorps conjugués au fluorochrome APC ou aux fluorochromes tandems APC-Cy7, APC-H7 ou APC-Alexa 750.

Les matériaux et les méthodes utilisés sont les suivants :

### Matériaux :

Les anticorps anti-CD45, anti-CD20, anti-CD3, anti-CD19 conjugués au fluorochrome APC-Cy7 ou au fluorochrome APC-H7 ainsi que le tampon de lyse BD Phosflow Lyse/Fix Buffer 5X ont été achetés à BD Bioscience.

Le tampon de lyse était un tampon de lyse au chlorure d'ammonium.

La solution saline tamponnée phosphate (PBS) provenait de Biomérieux, le sérum bovin foetal (FBS) a été acheté à Pan Biotech Gmbh et chauffé pendant 30 minutes à 56°C avant utilisation. L'acide L-ascorbique (vitamine C) provenait de SIGMA-ALDRICH.

### Méthode :

Des aliquots de sang périphérique humain (contenant 500 000 leucocytes) ont été incubées dans le tampon de lyse puis lavées trois fois avec du PBS 1X supplémenté avec 2,5% de FBS. Les cellules (500 000) ont ensuite été immuno-marquées avec 5µl de chacun des anticorps voulus conjugués soit au tandem APC voulu soit à APC seul dans 100µl de tampon d'incubation pendant 20 mn à température ambiante, et lavées trois fois avec la solution de PBS/FBS et remises en suspension dans 200µl de solution PBS/FBS.

Les cellules mortes ont été marquées pour leur exclusion lors de l'analyse des données acquises par cytométrie en flux en utilisant l'agent intercalant 7-aminoactinomycine-D (7-AAD) de SIGMA-ALDRICH.

Pour chaque expérience d'immuno-marquage, les cellules ont été mélangées et soumises à une analyse par cytométrie en flux sur un appareil FACSCanto II de Becton Dickinson. De 10 000 à 50 000 évènements étaient acquis par test. Les cellules singulets ont été sélectionnées par une procédure de tri cellulaire communément utilisée : intersection des trois fenêtres (P1, P2 et P3) des histogrammes biparamétriques.

Pour chaque type de cellules, le pourcentage de découplage a été calculé comme précédemment décrit en utilisant le logiciel BD FACSDIVA.

Les figures annexées montrent une expérience représentative de trois à quatre expériences répétées.

Tout d'abord, les inventeurs ont vérifié que la dégradation des fluorochromes tandem APC-Cy7 et APC-H7 conjugués à des anticorps dirigés vers des protéines de surface des cellules se produisait dans le modèle cellulaire décrit ci-dessus en provoquant l'apparition d'un signal dans le canal APC.

Pour cela, des cellules de sang périphérique humain ont été marquées avec des anticorps anti-CD45 conjugués soit au tandem APC-Cy7 soit au tandem APC-H7.

Les cellules ont été analysées par cytométrie en flux immédiatement après la préparation de la solution contenant les cellules pour analyse par cytométrie en flux, c'est-à-dire au temps T0, et 30 minutes plus tard, c'est-à-dire au temps T30.

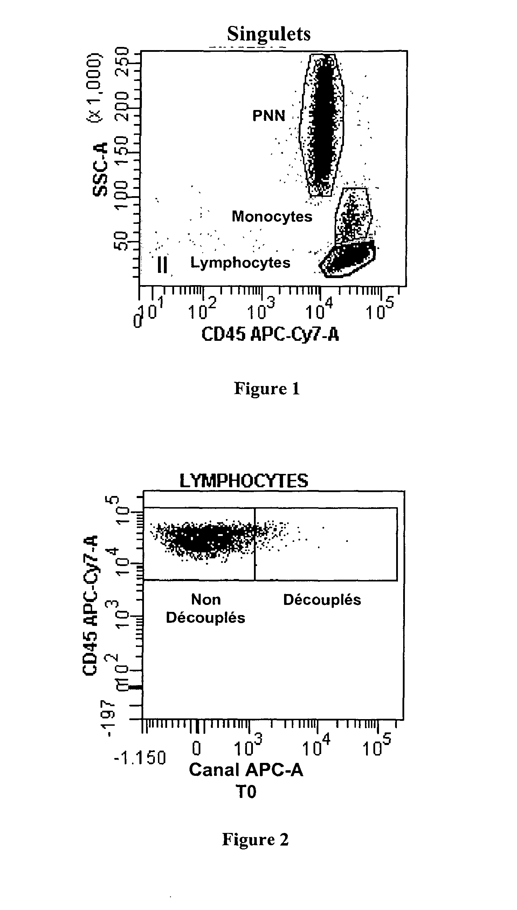

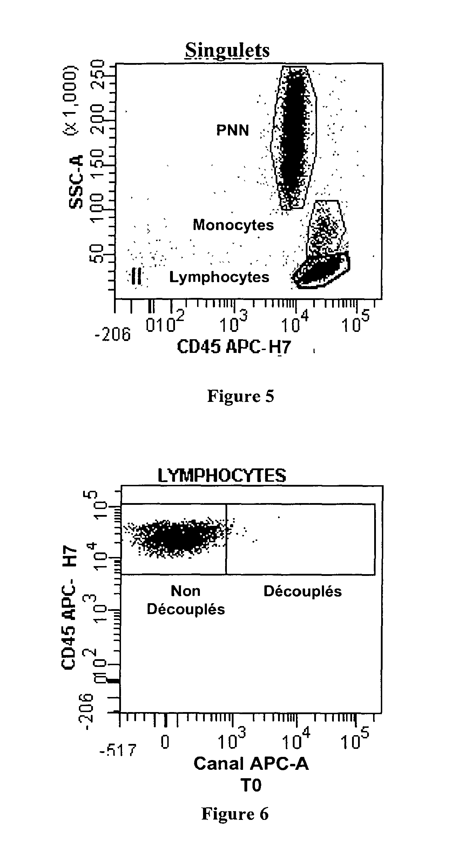

La figure 1 et la figure 5, montrent que, comme attendu, les anticorps anti-CD45 pan-leucocyte conjugués aux fluorochromes tandems APC marquent les lymphocytes, les monocytes et les leucocytes polynucléaires neutrophiles.

Dans ces deux figures, le terme « PNN » signifie « leucocyte ou cellule polynucléaire neutrophile ».

La population des lymphocytes marquée avec un anticorps anti-CD45 conjugué soit au tandem APC-Cy7 soit au tandem APC-H7, a été sélectionnée et des tracés bidimensionnels représentant l'intensité de leur fluorescence observée dans le canal APC et dans le canal APC-Cy7, au temps T0 et au temps T30 ont été générés.

Ces tracés sont montrés aux figures 2, 3, 5 et 7.

On voit à partir des figures 2 et 5 que, au temps T0, les lymphocytes apparaissent sous la forme d'une population cellulaire uniforme, qui est hautement positive dans le canal APC-Cy7 ou APC-H7 et faiblement positive dans le canal APC.

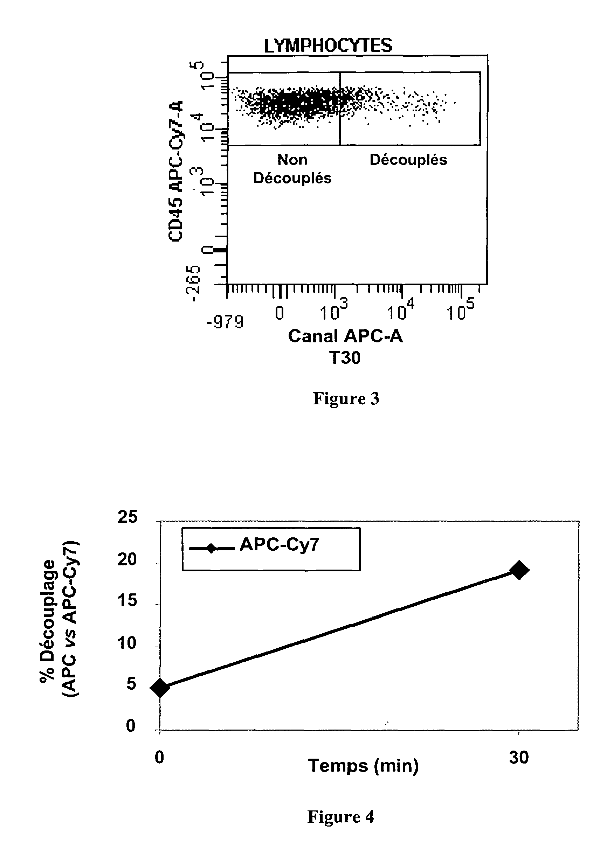

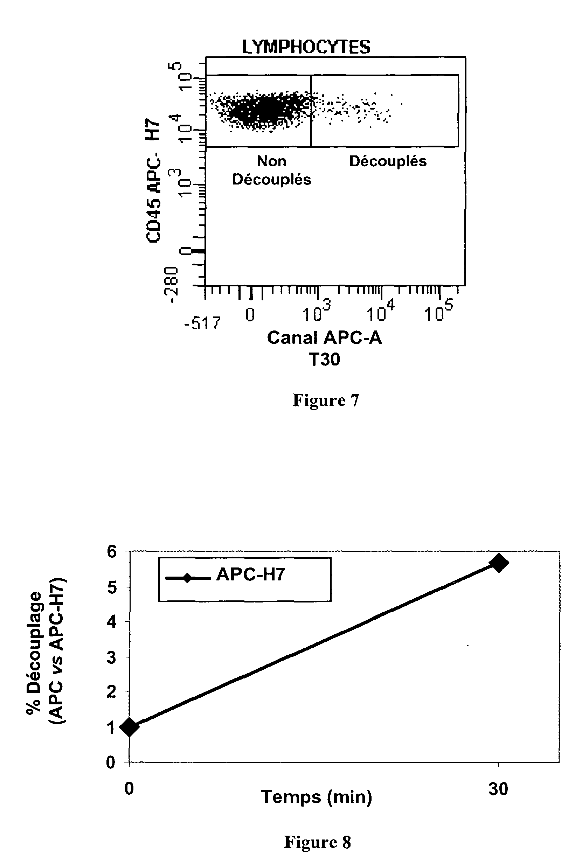

En contraste, on voit à partir des figures 3 et 7, qu'après une incubation de 30 minutes à température ambiante et dans le noir, la population cellulaire des lymphocytes se répand dans le canal APC.

En considérant les intensités de fluorescence médianes (MFI), on observe une augmentation du signal APC au temps T30 en comparaison au temps initial T0 parmi les cellules positives à CD45 conjuguées au tandem APC-Cy7 ou au tandem APC-H7.

Ensemble, ces observations suggèrent que le signal augmenté dans le canal APC est relié à une altération des fluorochromes tandems APC.

Pour évaluer ce phénomène, les inventeurs ont ciblé une sous-population émettant dans le canal APC, c'est-à-dire positive à APC, dans la population de lymphocytes positifs au tandem APC-Cy7 ou APC-H7, c'est-à-dire émettant dans le canal APC-Cy7 ou APC-H7, c'est-à-dire les populations indiquées « découplé » en figure 3 et 7.

Le seuil de positivité a été déterminé dans le canal APC au temps T0 et reporté au temps T30. Le pourcentage de cellules "découplées" a été exprimé comme le rapport des nombres totaux des cellules positives à la fois à APC et au tandem APC sur le total des lymphocytes positifs au tandem APC.

Les résultats sont représentés en figures 4 et 8.

Comme on le voit sur ces figures, le pourcentage de découplage des fluorochromes APC-Cy7 et APC-H7 est plus élevé au temps T30 qu'au temps T 0.

Des résultats similaires ont été obtenus avec des anticorps anti-CD45 couplés au tandem APC-Alexa 750.

Le pourcentage de base de découplage (découplage au temps TO) est plus élevé pour le fluorochrome tandem APC-Cy7 que pour le fluorochrome tandem APC-H7 mais les rapports de découplage des deux fluorochromes tandems APC ont une variation similaire pendant le temps d'incubation, c'est-à-dire une augmentation de quatre à cinq fois, comme on le voit en figure 4 et 8.

Ensemble, ces données suggèrent que les fluorochromes tandems APC conjugués à des anticorps anti-CD45 sont soumis à une dégradation dans le temps dans les lymphocytes.

Cette première partie de l'étude a permis de vérifier que dans les conditions expérimentales utilisées, le phénomène de découplage observé lors d'une analyse par cytométrie en flux utilisant les tandems APC, c'est-à-dire les tandems APC-Cy7, APC-H7 ou APC-Alexa 750, se produit bien. Cela valide le modèle utilisé.

Ensuite, les inventeurs ont étudié l'influence du contexte cellulaire sur ce phénomène de découplage.

Pour cela, ils ont analysé la stabilité des fluorochromes tandems APC liés à des lymphocytes et l'ont comparé à la stabilité de ces mêmes tandems APC liés à des billes. Cette comparaison permet de déterminer si oui ou non c'est le tandem APC qui est dégradé, quel que soit le contexte dans lequel il est utilisé, ou si le tandem APC est dégradé seulement en présence de cellules.

Les billes utilisées dans ces expériences étaient des billes Compbeads^{®} de Becton Dickinson.

Les lymphocytes, comme les billes, ont été marqués avec des anticorps anti-CD45 conjugués au fluorochrome tandem APC-Cy7 ou au fluorochrome tandem APC-H7.

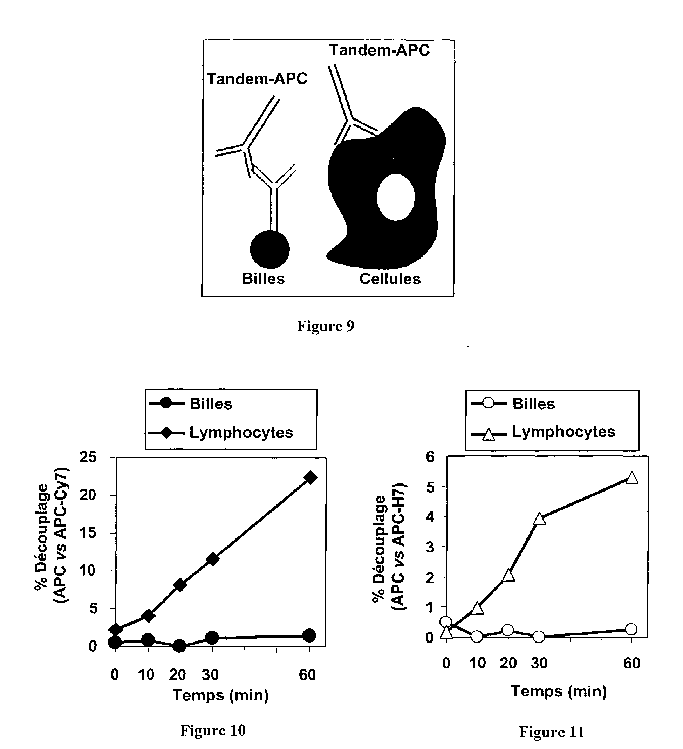

Les figures 9 à 15 représentent les données acquises lors de ces expériences.

Plus précisément, la figure 9 montre, sous forme schématique, la liaison des anticorps soit aux billes (billes Compbeads^{®} de Becton Dickinson) soit aux lymphocytes.

Comme on le voit en figure 9, les anticorps sont liés aux marqueurs cellulaires par leur site de liaison d'antigène tandis que les billes sont liées aux marqueurs via la reconnaissance des chaînes légères.

L'évolution du pourcentage de découplage observé en fonction du temps lorsque le tandem APC-Cy7 est lié soit aux billes (billes Compbeads^{®} de Becton Dickinson) soit aux lymphocytes est représentée en figure 10, et l'évolution du pourcentage de découplage observée en fonction du temps lorsque les billes (billes Compbeads^{®} de Becton Dickinson) ou les lymphocytes sont liés au tandem APC-H7 sont représentées en figure 11.

On voit à partir de ces figures que de façon très intéressante, le pourcentage de découplage des fluorochromes APC-Cy7 et APC-H7 augmente dans le temps pour les anticorps liés aux cellules, seulement. En effet, aucun découplage n'est observé pour les anticorps couplés aux billes.

Des résultats similaires ont été obtenus avec d'autres anticorps tels que l'anticorps anti-CD20.

Ces données suggèrent que l'altération des fluorochromes tandems APC est liée au marquage des cellules plutôt qu'à une instabilité intrinsèque des fluorochromes dans les conditions classiques d'utilisation de ces fluorochromes.

Pour vérifier que la dégradation des fluorochromes tandems APC se produisait quelque soit l'anticorps utilisé, plusieurs anticorps dirigés vers des marqueurs de membranes de lymphocytes et conjugués soit au fluorochrome tandem APC-Cy7 soit au fluorochrome tandem APC-H7 ont été testés.

Pour cela, les fluorochromes tandems APC-Cy7 et APC-H7 conjugués à des anticorps dirigés vers les lymphocytes B, anti-CD20 ou anti-CD19, ou les marqueurs des lymphocytes T, anti-CD3, ont été utilisés.

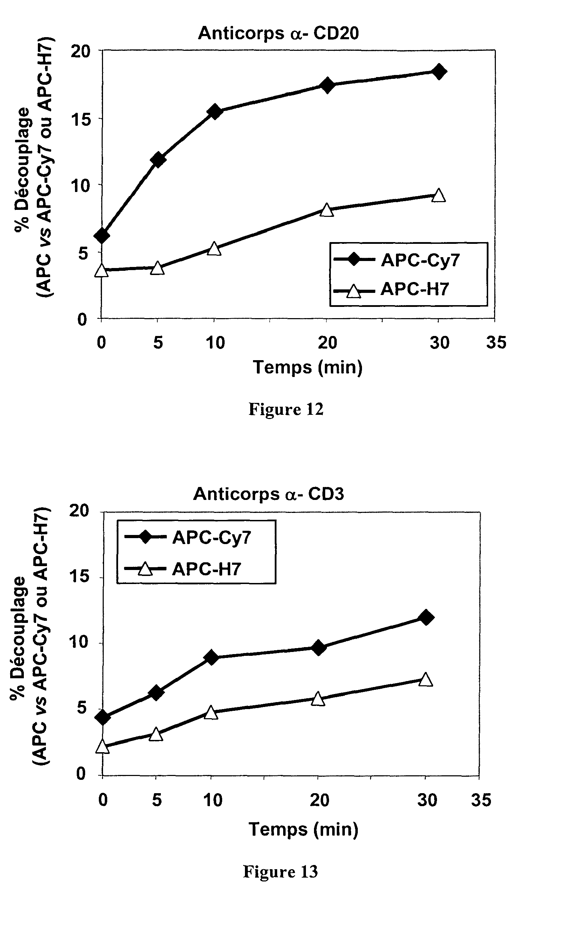

La figure 12 montre l'évolution du pourcentage de découplage en fonction du temps d'incubation, observé lors de l'utilisation des anticorps anti-CD20 conjugués soit au fluorochrome tandem APC-Cy7 soit au fluorochrome tandem APC-H7. La figure 13 montre l'évolution du pourcentage de découplage, en fonction du temps d'incubation, lors de l'utilisation d'anticorps anti-CD3 conjugués au fluorochrome tandem APC-Cy7 ou tandem APC-H7.

Comme on le voit en figures 12 et 13, on observe un phénomène de découplage de plus en plus important en fonction du temps d'incubation.

Une fois encore, les niveaux de dégradation du fluorochrome tandem APC-Cy7 sont plus élevés que ceux du fluorochrome tandem APC-H7.

Ces résultats confirment également que l'altération des fluorochromes tandems APC se produit aussi bien lors du ciblage des marqueurs de surface des lymphocytes T que des lymphocytes B.

L'influence du type de cellules a également été étudiée.

Pour cela, des populations de différents types de cellules leucocytes ont été marqués avec des anticorps anti-CD45 pan-leucocytes conjugués soit au fluorochrome tandem APC-Cy7 soit au fluorochrome tandem APC-H7 et le découplage dans chacune des sous-populations a été déterminé dans le temps.

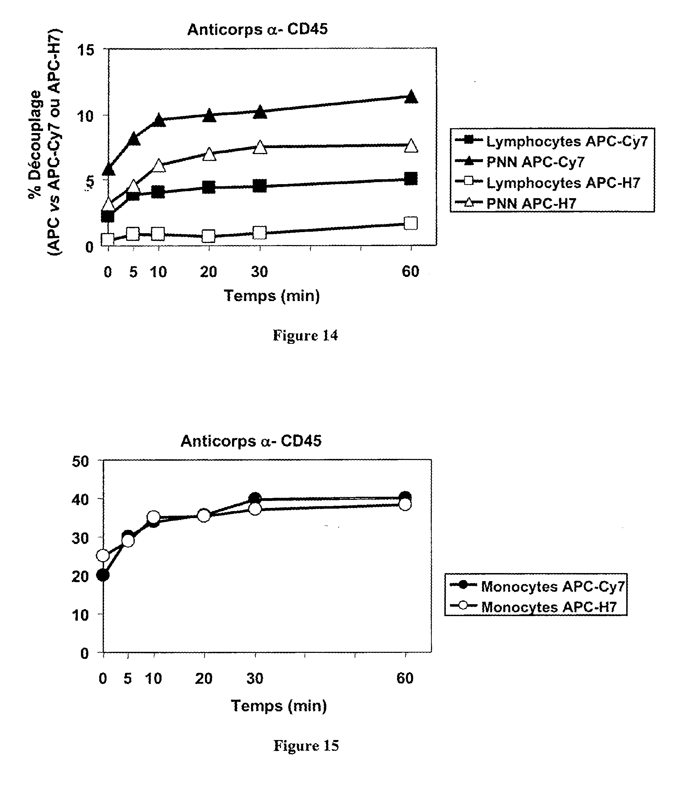

Les résultats obtenus sont représentés en figure 14 qui montre le pourcentage de découplage en fonction du temps :
- de lymphocytes marqués par des anticorps anti-CD45 conjugués au tandem APC-Cy7,
- de cellules polynucléaires neutrophiles marquées par des anticorps anti-CD45 conjugués au fluorochrome tandem APC-Cy7,
- de lymphocytes marqués par des anticorps anti-CD45 conjugués au fluorochrome tandem APC-Cy7,
- de lymphocytes marqués par des anticorps anti-CD45 conjugués au fluorochrome tandem APC-H7, et
- de cellules polynucléaires neutrophiles marquées par des anticorps anti-CD45 conjugués au tandem APC-H7, et
   en figure 15 qui montre le pourcentage de découplage observé sur des monocytes marqués par des anticorps anti-CD45 conjugués soit au fluorochrome APC-Cy7 soit au fluorochrome APC-H7.

Comme on le voit dans ces figures, le pourcentage de découplage des fluorochromes tandems APC-Cy7 et APC-H7 augmente lorsque les anticorps anti-CD45 sont liés aussi bien à des lymphocytes qu'à des monocytes et qu'à des granulocytes.

Cependant, pour tous les tandems APC conjugués, le taux de dégradation augmente graduellement entre les lymphocytes, les monocytes et les granulocytes.

Ces résultats montrent que la dégradation des fluorochromes tandems APC dépend du type cellulaire mais se produit dans tous les cas.

En première conclusion, les expériences ci-dessus des inventeurs montrent que le phénomène de découplage n'apparaît qu'en présence de cellules, quel que soit ce type de cellules et qu'il y a un phénomène de découplage plus ou moins important selon le type de cellules.

Sur la base de ces résultats, les inventeurs ont alors cherché les voies possibles pour bloquer la dégradation dépendante des cellules des fluorochromes tandems APC.

Pour cela, tout d'abord, des cellules marquées avec des anticorps anti-CD45 couplés aux fluorochromes tandems APC ont été fixées grâce à un tampon BD Phosflow Lyse/Fix Buffer avant la mesure de la dégradation du fluorochrome dans le temps.

Comme on le voit en figure 21, qui représente l'évolution du pourcentage de découplage dans le temps qui se produit avec des lymphocytes ou des cellules polynucléaires neutrophiles marqués par des anticorps anti-CD45 couplés au fluorochrome tandem APC-Cy7 fixés ou non fixés, le pourcentage de découplage se produisant reste stable dans le temps et proche de la ligne de base, c'est-à-dire de la valeur obtenue au temps T0, dans les lymphocytes et les leucocytes polynucléaires neutrophiles. Il en est de même dans les monocytes, comme on le voit en figure 23 qui représente l'évolution du pourcentage de découplage se produisant pour des monocytes liés à des anticorps anti-CD45 conjugués au fluorochrome tandem APC-Cy7, en fonction du temps.

Les mêmes résultats ont été obtenus sur ces mêmes types de cellules en utilisant des anticorps anti-CD45 couplés cette fois au fluorochrome tandem APC-H7.

L'inhibition forte du phénomène de découplage en utilisant un tampon de fixation, qui stoppe l'activité métabolique des cellules, démontre le rôle du métabolisme cellulaire dans l'altération des fluorochromes tandem APC.

Mais la fixation des cellules entraîne la mort des cellules et surtout peut entraîner la perte d'épitopes fonctionnels

De plus, la fixation des cellules n'est pas une des conditions de mise en oeuvre du protocole d'analyse des cellules en routine.

Ainsi, bien que la fixation des cellules permette d'inhiber le phénomène de découplage, il ne s'agit pas d'une méthode préférée de l'invention.

Les inventeurs ont alors étudié dans des conditions similaires de marquage de leucocytes, l'effet de la température en effectuant une incubation à 4°C. Pour mémoire, dans les essais de routine, cette incubation se pratique à température ambiante, c'est-à-dire entre 18°C et 25°C inclus.

Les résultats sont montrés aux figures 22 et 24.

La figure 22 représente l'évolution du pourcentage de découplage se produisant dans le temps lors de l'analyse par cytométrie en flux, de lymphocytes incubés à température ambiante et de lymphocytes incubés à 4°C, de cellules polynucléaires neutrophiles incubées à température ambiante, et de cellules polynucléaires neutrophiles incubées à 4°C.

La figure 24 représente l'évolution du pourcentage de découplage apparaissant lors de l'analyse par cytomérie en flux de monocytes marqués avec un anticorps anti-CD45 conjugués au fluorochrome tandem APC-Cy7, incubés à température ambiante et de monocytes incubés à 4°C.

Comme on le voit à partir des figures 22 et 24, à 4°C, tous les pourcentages de dégradation du fluorochrome tandem APC-Cy7 dans les lymphocytes, les leucocytes polynucléaires neutrophiles et les monocytes restent très bas sans augmentation dans le temps en comparaison aux pourcentages observés sur ces mêmes cellules dans les mêmes conditions mais avec une incubation à température ambiante.

Les mêmes résultats ont été obtenus en utilisant le fluorochrome tandem APC-H7.

Ces expériences confirment, une fois encore, l'importance du métabolisme cellulaire sur la dégradation des fluorochromes tandems APC.

Mais, ici aussi l'incubation à 4°C n'est pas une condition du protocole d'analyse de routine des cellules. De plus, pour une incubation à 4°C, la durée d'incubation est plus longue et nécessite des conditions plus contraignantes. Cette méthode n'est donc pas une méthode préférée de l'invention, bien qu'efficace.

Les inventeurs ont alors, pour bloquer l'activité métabolique des cellules, lorsque les cellules sont maintenues à température ambiante, testé l'effet de l'azide de sodium (NaN₃) qui est souvent ajouté à de nombreux conjugués.

On a alors observé que 0,05% de NaN₃ n'affectait que faiblement le phénomène de découplage avec des lymphocytes mais que 0,05% de NaN₃ inhibait fortement l'altération des fluorochromes tandems APC-H7.

Des effets similaires ont été observés avec des monocytes et des leucocytes polynucléaires neutrophiles sans affecter la viabilité cellulaire.

Ceci démontre qu'inhiber l'activité métabolique mène à une protection des fluorochromes tandems APC.

Mais l'azide de sodium inhibe de façon irréversible la cytochrome oxidase et est considéré comme toxique pour le manipulateur et l'environnement.

Les inventeurs ont alors, et ce contrairement au préjugé existant dans l'art, étudié l'effet d'une exposition à la lumière sur la dégradation des fluorochromes tandems APC.

En effet, selon l'art antérieur, d'une manière générale, et ce pour tous les fluorochromes, il est conseillé d'effectuer le marquage des cellules à l'abri de la lumière et pour les fluorochromes tandem-APC, ceci est particulièrement mis en avant dans les instructions du fabricant.

Les inventeurs ont donc remis en cause ce préjugé et ont exposé des cellules sanguines immuno-marquées à une source de lumière artificielle (tube au néon) et ont comparé le pourcentage de découplage obtenu dans ces conditions au pourcentage de découplage obtenu lorsque l'immuno-marquage était effectué à l'abri de la lumière.

Les résultats obtenus sont représentés aux figures 16 à 20.

La figure 16 représente l'évolution du pourcentage de découplage se produisant pour des lymphocytes, des monocytes, et des cellules polynucléaires neutrophiles marquées par l'anticorps anti-CD45 conjugué au fluorochrome tandem APC-Cy7, en fonction du temps et lors d'une exposition à la lumière ou sans exposition à la lumière.

La figure 17 représente l'évolution du pourcentage de découplage obtenu sur ces mêmes cellules mais marquées par l'anticorps anti-CD45 conjugués au fluorochrome tandem APC-H7, également en présence de lumière ou en l'absence de lumière.

Comme on le voit à partir des figures 16 et 17, de façon surprenante, le pourcentage de dégradation des fluorochromes tandems APC-Cy7 et APC-H7, pour ces trois types de cellules était similaire en présence ou en l'absence de lumière sur une courte période de temps de 1 h. Cette absence d'un effet de la lumière sur la dégradation de ces tandems dans ces conditions expérimentales s'accompagne d'un blanchiment équivalent des fluorochromes APC et des fluorochromes tandems APC-Cy7 ou APC-H7.

Cette hypothèse a été confirmée en immuno-marquant des billes, c'est-à-dire en l'absence de cellules, avec les anticorps anti-CD45 conjugués au fluorochrome tandem APC-Cy7, au fluorochrome tandem APC-H7 et au fluorochrome APC.

L'évolution des intensités moyennes de fluorescence (MFI) en fonction du temps sont représentées aux figures 18, 19 et 20.

Plus précisément, la figure 18 représente l'évolution de l'intensité moyenne de fluorescence (MFI), en fonction du temps, de billes en présence et en absence de lumière, immuno-marquées avec des anticorps anti-CD45 conjugués au tandem APC-Cy7.

La figure 19 représente l'évolution de l'intensité moyenne de fluorescence (MFI) en fonction du temps de billes marquées avec des anticorps anti-CD45 conjugués au tandem APC-H7, en présence et en absence de lumière.

La figure 20 montre l'évolution des intensités moyennes de fluorescence (MFI) en fonction du temps de billes marquées avec des anticorps anti-CD45 conjugués au fluorochrome APC, en présence et en absence de lumière.

Comme on le voit à partir de ces figures, les intensités moyennes de fluorescence des trois fluorochromes diminuent dans le temps graduellement avec des cinétiques similaires en l'absence ou en présence de lumière, suggérant que l'absence de l'effet de la lumière n'est pas reliée à une sous-estimation du blanchiment du fluorochrome APC.

Ensemble, ces expériences écartent fortement l'hypothèse selon laquelle une exposition à la lumière jouerait un rôle en tant que catalyseur de la dégradation des tandems APC dans ces conditions expérimentales.

Les inventeurs ont ainsi surmonté un préjugé dans l'art.

Ils ont alors exploré le rôle potentiel d'un phénomène d'oxydation.

Les inventeurs ont alors décidé d'étudier l'influence éventuelle de la présence de radicaux libres et/ou d'un phénomène d'oxydation sous jacent à la dégradation des fluorochromes tandems APC.

En effet, les résultats obtenus précédemment montrent que la dégradation des fluorochromes tandems APC se produisait en proportion plus élevée dans les monocytes que dans les lymphocytes et les cellules polynucléaires neutrophiles, ce qui suggère que le phénomène de découplage pourrait dépendre des propriétés du type de cellules étudiées.

Une des fonctions principales des monocytes et des macrophages est la phagocytose et la destruction des microorganismes, qui dépendent de la production de superoxyde et de peroxyde d'hydrogène (H₂O₂).

Pour tester l'influence de H₂O₂ sur la dégradation des fluorochromes tandems APC, les inventeurs ont immuno-marqué des cellules avec un anticorps anti-CD45 conjugué à APC-Cy7 et les ont incubé avec ou sans H₂O₂.

Les cellules ont été analysées pendant 20 à 60 minutes.

Les résultats obtenus montrent que 0,01 mM d'H₂O₂ ne modifie pas le pourcentage de découplage chez les lymphocytes, tandis que 0,1 mM d'H₂O₂ l'augmentait.

Un rôle comparable du peroxyde d'hydrogène a été observé avec les monocytes et les cellules polynucléaires neutrophiles, sans aucun effet sur la viabilité des cellules, suggérant que la présence de radicaux libres et/ou un phénomène d'oxydation pouvaient jouer un rôle dans le phénomène de découplage.

Il faut noter, que dans le temps, le peroxyde d'hydrogène pouvait induire le découplage des anticorps conjugés avec les tandems APC-Cy7 liés à des billes.

En raison des multiples inconvénients de l'utilisation d'une fixation de cellules, ou d'une faible température ou d'azide de sodium pour inhiber la dégradation des fluorochromes tandems APC, les inventeurs ont testé l'utilisation d'un agent anti-oxydant et/ou piégeant les radicaux libres sur le phénomène de découplage.

Pour cela, des cellules immuno-marquées avec un anticorps anti-CD45 conjugué à un tandem APC ont été incubées avec ou sans vitamine C, utilisée en tant qu'agent antioxydant et/ou piégeant les radicaux libres, bien que la vitamine C soit elle-même très fragile en solution et dégradée par l'air, la lumière et la chaleur, avant incubation à température ambiante. Les résultats sont représentés aux figures 25 et 26.

La figure 25 représente l'évolution du pourcentage de découplage en fonction du temps se produisant dans des lymphocytes marqués par des anticorps anti-CD45 conjugués au tandem APC-Cy7 en l'absence de vitamine C et en présence de vitamine C à une concentration finale 1 millimolaire (1mM), ainsi que de cellules polynucléaires neutrophiles marquées par des anticorps anti-CD45 conjugués au tandem APC-Cy7 en l'absence de vitamine C et en présence de vitamine C à une concentration finale 1mM.

La figure 26 représente l'évolution du pourcentage de découplage obtenu sur des monocytes liés à l'anticorps anti-CD45 conjugué au tandem APC-Cy7 en l'absence de vitamine C et en présence de vitamine C à une concentration finale 1mM.

Le pourcentage de dégradation a été suivi pendant 1 heure.

Comme on le voit à partir des figures 25 et 26, de façon surprenante, la présence de vitamine C inhibe fortement le pourcentage de découplage dans le temps dans les lymphocytes, les leucocytes polynucléaires neutrophiles et dans une moindre mesure dans les monocytes.

Ce résultat montre un rôle important de l'oxydation et/ou de la présence de radicaux libres dans l'altération des conjugués tandems APC sur des cellules immuno-marquées, oxydation et/ou présence de radicaux libres qui de façon surprenante est inhibée par la vitamine C en solution.

Ce rôle a été confirmé en soumettant des billes Compbeads^{®} à des concentrations croissantes de H₂O₂ (de 0 à 1 mM) avec ajout de 1 mM de vitamine C. Cet ajout de vitamine C bloque l'altération du signal du tandem APC à toutes les concentrations testées de H₂O₂.

Ainsi, la vitamine C est utilisée très avantageusement pour empêcher un signal anormal dans le canal APC.

Dès lors, l'invention repose sur la découverte du mécanisme d'altération des tandems APC sur des cellules sanguines immuno-marquées, c'est-à-dire un mécanisme d'oxydation et/ou dégradation par les radicaux libres des fluorochromes tandem APC et non par un phénomène de photo-dégradation, comme cela était crû dans l'art antérieur.

L'invention repose également sur la découverte que la vitamine C exerce efficacement son action antioxydante et/ou de piège de radicaux libres même lorsque qu'elle est en solution pendant une période de temps suffisant pour permettre l'analyse.

Bien que dans ce qui précède on ait décrit l'invention appliquée à des cellules sanguines, il apparaîtra clairement à l'homme de l'art qu'elle est applicable à tous types de cellules analysables par cytométrie en flux, telles que des cellules de moelle ou encore des cellules de plantes, etc.

De la même façon, bien que l'utilisation de la vitamine C a permis de découvrir le mécanisme donnant apparition au phénomène de découplage, il apparaîtra clairement à l'homme de l'art que tout agent antioxydant et/ou piégeant les radicaux libres non toxique pour les cellules peut être utilisé dans l'invention.

En particulier, l'acide ascorbique, sous toutes ses formes est particulièrement adapté.

Cependant, l'utilisation de l'acide ascorbique sous sa forme L, c'est-à-dire la vitamine C, a de nombreux avantages par rapport à d'autres agents anti-oxydants et/ou piégeant les radicaux libres : elle est soluble en milieu aqueux et elle n'est pas toxique pour les cellules à ces concentrations.

De même, bien que les essais décrits et illustrés aient été effectués avec une concentration finale en vitamine C de 1mM, des essais ont également été effectués à des concentrations en présence de vitamine C à une concentration 0,1 millimolaire (0,1mM) et 10 millimolaires (10mM).

Lorsque les cellules sont des lymphocytes, à une concentration 0,1mM, la vitamine C produit déjà un effet mais diminué. Ainsi, des concentrations inférieures de vitamine C ne sont pas adaptées pour obtenir une baisse suffisante du pourcentage de découplage. De plus, au-delà de 10 mM, la morphologie et la viabilité des lymphocytes sont modifiées.

Les meilleurs résultats sont obtenus à des concentrations en vitamine C comprises entre 1 mM et 5 mM inclus, avec les lymphocytes.

Dès lors, la concentration préférée, dans l'invention, de vitamine C, avec des lymphocytes est de 1 mM car elle permet d'obtenir une inhibition du phénomène de découplage optimale avec une concentration en vitamine C optimale.

Lorsque les cellules sont des monocytes, une baisse suffisante du pourcentage de découplage apparaît pour une concentration en vitamine C de 1 mM. Cette baisse est présente jusqu'à une concentration en vitamine C de 10 mM. Au-delà de cette concentration de 10 mM la viabilité et la morphologie des monocytes sont modifiées.

Lorsque les cellules sont des cellules polynucléaires neutrophiles, une baisse suffisante du pourcentage de découplage est obtenue avec une concentration en vitamine C de 0,25 mM.

Cette baisse est présente jusqu'à une concentration en vitamine C de 10 mM. Au-delà de 10 mM, les cellules commencent à subir une diminution de leur viabilité.

Les meilleurs résultats sont obtenus avec une concentration en vitamine C comprise entre 0,75 mM et 4 mM inclus, pour les cellules polynucléaires neutrophiles. Au-delà de 4 mM, les cellules commencent à subir une altération morphologique.

Pour cette raison, la concentration plus préférée en vitamine C est comprise entre 0,75 mM et 2 mM inclus, et la plus préférée est de 0,75 mM pour les cellules polynucléaires neutrophiles.

De plus, alors que dans ce qui précède l'agent antioxydant et/ou piégeant les radicaux libres a été ajouté au moment de l'incubation dans la solution de marquage, il apparaîtra clairement à l'homme de l'art qu'il peut être ajouté à l'avance, par exemple dans le tampon d'incubation préparé à l'avance et utilisé pour le marquage avec les anticorps conjugués aux fluorochromes

Dès lors, l'invention concerne l'utilisation d'un agent antioxydant et/ou piégeant les radicaux libres, de préférence de l'acide ascorbique et le plus préférablement de l'acide ascorbique de forme L, ou vitamine C, dans un procédé d'analyse en cytométrie de flux.

De préférence, la vitamine C est utilisée à une concentration finale dans la solution de marquage comprise entre 0,1mM et 10mM inclus, de préférence 1 mM.

De la même façon, l'invention concerne également un tampon d'incubation utilisé lors du marquage des cellules qui comprend un agent antioxydant et/ou piégeant les radicaux libres, de préférence de l'acide ascorbique, et le plus préférablement de la vitamine C.

## Revendications

1. Procédé d'analyse de cellules par cytométrie en flux comprenant une étape de marquage des cellules dans une solution de marquage comprenant un tampon d'incubation et le ou les anticorps de marquage conjugués à au moins un fluorochrome qui est un tandem APC **caractérisé en ce qu'**un agent antioxydant et/ou piégeant les radicaux libres est introduit dans la solution de marquage, pour inhiber la dégradation dudit au moins un fluorochrome.

2. Procédé selon la revendication 1 **caractérisé en ce que** les cellules sont des cellules sanguines.

3. Procédé selon la revendication 1 ou 2 **caractérisé en ce que** l'agent antioxydant et/ou piégeant les radicaux libres est l'acide ascorbique.

4. Procédé selon l'une quelconque des revendications précédentes **caractérisé en ce que** l'agent antioxydant et/ou piégeant les radicaux libres est la vitamine C.

5. Procédé selon l'une quelconque des revendications précédentes **caractérisé en ce que** les cellules sont des lymphocytes et **en ce que** l'agent antioxydant et/ou piégeant les radicaux libres est la vitamine C présente dans la solution de marquage à une concentration finale comprise entre 0,1 à 10mM inclus, de préférence comprise entre 1 mM à 5 mM inclus.

6. Procédé selon la revendication 5 **caractérisé en ce que** la vitamine C est présente dans la solution de marquage à une concentration de 1 mM.

7. Procédé selon l'une quelconque des revendications 1 à 4 **caractérisé en ce que** les cellules sont des monocytes et **en ce que** l'agent anti-oxydant et/ou piégeant les radicaux libres est la vitamine C présente dans la solution de marquage à une concentration finale comprise entre 1 mM à 10 mM inclus, de préférence de 1 mM.

8. Procédé selon l'une quelconque des revendications 1 à 4 **caractérisé en ce que** les cellules sont des cellules polynucléaires neutrophiles et **en ce que** l'agent anti-oxydant et/ou piégeant les radicaux libres est la vitamine C présente dans la solution de marquage à une concentration finale comprise entre 0,25 mM à 10 mM inclus, de préférence comprise entre 0,25 mM et 4 mM inclus, plus préférablement comprise entre 0,25 mM et 2 mM inclus, le plus préférablement de 0,75 mM.

9. Utilisation d'un agent antioxydant et/ou piégeant les radicaux libres pour inhiber la dégradation des fluorochromes tandems APC-Cy7, APC-H7 et APC- Alexa 750 lors de l'analyse par cytométrie en flux de cellules.

10. Utilisation selon la revendication 9 **caractérisée en ce que** les cellules sont des cellules sanguines.

11. Utilisation selon la revendication 9 ou 10 **caractérisée en ce que** l'agent antioxydant et/ou piégeant les radicaux libres est l'acide ascorbique.

12. Utilisation selon l'une quelconque des revendications 9 à 11 **caractérisée en ce que** l'agent antioxydant et/ou piégeant les radicaux libres est la vitamine C.

13. Utilisation selon l'une quelconque des revendications 9 à 12 **caractérisée en ce que** les cellules sont des lymphocytes et **en ce que** l'agent antioxydant et/ou piégeant les radicaux libres est la vitamine C présente dans la solution analysée par cytométrie en flux en une concentration comprise entre 0,1 et 10 mM inclus, de préférence comprise entre 1 mM et 5 mM inclus.

14. Utilisation selon l'une quelconque des revendications 9 à 12 **caractérisée en ce que** la vitamine C est présente dans la solution de cellules à analyser en une concentration de 1 mM.

15. Utilisation selon l'une quelconque des revendications 9 à 12 **caractérisée en ce que** les cellules sont des monocytes et **en ce que** l'agent anti-oxydant et/ou piégeant les radicaux libres est la vitamine C présente dans la solution de marquage à une concentration finale comprise entre 1 mM à 10 mM inclus, de préférence de 1 mM.

16. Utilisation selon l'une quelconque des revendications 9 à 12 **caractérisée en ce que** les cellules sont des cellules polynucléaires neutrophiles et **en ce que** l'agent anti-oxydant et/ou piégeant les radicaux libres est la vitamine C présente dans la solution de marquage à une concentration finale comprise entre 0,25 mM à 10 mM inclus, de préférence comprise entre 0,25 mM et 4 mM inclus, plus préférablement comprise entre 0,25 mM et 2 mM inclus, le plus préférablement de 0,75 mM.

17. Tampon d'incubation **caractérisé en ce qu'**il comprend un agent antioxydant et/ou piégeant les radicaux libres.

18. Tampon selon la revendication 17 **caractérisé en ce que** l'agent antioxydant et/ou piégeant les radicaux libres est l'acide ascorbique.

19. Tampon selon la revendication 17 ou 18 **caractérisé en ce que** l'agent antioxydant et/ou piégeant les radicaux libres est la vitamine C.
